# EUROPEAN PATENT SPECIFICATION

(11) **EP 0 413 988 B1**
(45) Date of publication and mention of the grant of the patent: **06.05.1998**
(21) Application number: 90114322.2
(22) Date of filing: 26.07.1990
(51) Int. Cl.: A61B 1/00

(54) **Endoscope examination apparatus**
Endoskopisches Untersuchungsgerät
Appareil d'examen à endoscope

(30) Priority: 26.07.1989 JP 194756/89; 01.08.1989 JP 201012/89; 26.09.1989 JP 251419/89; 26.09.1989 JP 251417/89
(43) Date of publication of application: 27.02.1991
(73) Proprietor: OLYMPUS OPTICAL CO., LTD., Tokyo 151 (JP)
(72) Inventor: Nishikori, Toshiaki, Sagamihara-shi, Kanagawa-ken (JP); Nakajima, Yukio, c/o Olympus Optical Co. G.m.b.H., D-2000 Hamburg 1 (DE); Kawashima, Masahiro, Olympus Optical Co. G.m.b.H., D-2000 Hamburg 1 (DE); Greengrass, Stuart Malcom, c/o Keymed Ltd., Southend-on-Sea, Essex SS2 5QH (GB); Parker, Christopher, c/o Keymed Ltd., Southend-on-Sea, Essex SS2 5QH (GB); Takayama, Shuichi, Hachioji-shi, Tokyo (JP); Sanagi, Kenichiro, Mineola, New York 11501 (US); Nishigaki, Shinichi, Setagaya-ku, Tokyo (JP); Akui, Nobuaki, Hino-shi, Tokyo (JP); Yamaguchi, Tatsuya, Hino-shi, Tokyo (JP); Nakamura, Takeaki, Hino-shi, Tokyo (JP); Hayashi, Masaaki, Hachioji-shi, Tokyo (JP); Takano, Akira, Oume-shi, Tokyo (JP)
(74) Representative: Kahler, Kurt, Dipl.-Ing.

(56) References cited:
- DE-A- 2 749 703
- DE-A- 3 415 837
- DE-B- 3 743 920
- JP-U-63 143 301
- PATENT ABSTRACTS OF JAPAN vol. 13, no. 293 (C-615)(3641), 6 July 1989; & JP-A- 1086930 (TOSHIBA CORP.) 31.03.1989

## Description

### Background of the Invention

### Field of the Invention

The present invention relates to an endoscope examination apparatus having an endoscope examination bed as specified in the preamble of Claim 1. Such an apparatus is known e.g. from the Japanese Utility Model No. 63-143301.

### Related Art Statement

Recently, there has come into extensive use of an endoscope whereby the internal organs in the body cavity are observed by inserting an elongated insertable part into the body cavity, or whereby various treatments can be taken by using a treatment tool inserted into a treatment tool channel according to the demand.

In the case in which an endoscope is used, endoscope peripheral devices such as a light source device, an air and water supplying device and a high frequency cautery device are needed. These devices are usually stored in a cart or the like and are placed beside a bed so that an operator can operate these devices and/or so that an endoscope can be connected with these devices. However, if these peripheral devices are arranged on the same side of the bed as of the operator, these devices tend to become obstacles and hinder an endoscope examination.

In order to cope with this, a bed with attached devices needed for an endoscope examination has been proposed as shown in the Gazettes of Japanese Patent Laid Open Nos. 156461/1985, 83239/1989 and 86930/1989.

However, if various functions are included in a bed, it is inconvenient in that the bed becomes larger and electric devices are liable to get wet or dirty from the body fluid, dirt or the like of a patient.

Also, in the Gazette of Japanese Patent Laid Open No. 143301/1988, an apparatus is disclosed in that an operating panel of a video processor, which is a separate body from a bed, is removably provided to a bed. However, this apparatus has a problem in that a cable for connecting the operating panel and the video-processor is arranged apart from the bed without passing through the bed and therefore gets in the way.

Also, generally, since there is only one panel for operating said peripheral devices, it is troublesome for a team comprising the operator and nurses to operate the operating panel. Further, it is difficult to position the operating panel in accordance with the contents of the examination and the maneuverability of the panel is inconvenient.

Also, said peripheral devices are generally placed at a certain interval away from the operator and nurses so as not to hinder them. The operator and nurses connect an endoscope and treatment tools and so forth with said peripheral devices so that they can pursue an endoscope examination such as an insertion of an endoscope into the body cavity and ensuing observation, and/or carry out a treatment by using the treatment tools. Although such an endoscope examination is accompanied by an adjustment of a light source device and/or a high frequency burning device, the adjustment is done while operators are standing. Therefore, there is a problem in that the operator and nurses become quite exhausted if the endoscope examination lasts for several hours. There is also a problem in that the conducting of the examination is inconvenient because the peripheral devices are arranged at some distance from the operator and nurses.

Further, the endoscope and treatment tools can be taken out of a storage place and/or cart at the time of the endoscope examination. However, in the case in which an endoscope and treatment tools are taken out in this way, it is troublesome to carry them to a patient's bed and there is a possibility that the endoscope might hit another device or the like and be broken while it is being moved.

### Object and Summary of the Invention

A purpose of this invention is to provide an endoscope examination apparatus in which devices used for an endoscope examination are not included in an endoscope examination bed and can be easily controlled.

Another object of this invention is to provide an endoscope examination apparatus having readily operable devices used for an endoscope examination.

A further object of this invention is to provide an endoscope examination apparatus which can reduce the fatigue of a person who operates devices used for an endoscope examination.

Yet another object of this invention is to provide a workable endoscope examination apparatus in which it is not necessary to carry an endoscope and treatment tools.

An endoscope examination apparatus of this invention is defined in Claim 1.

The other features and advantages of this invention will be apparent from the following explanation.

### Brief Description of the Drawings

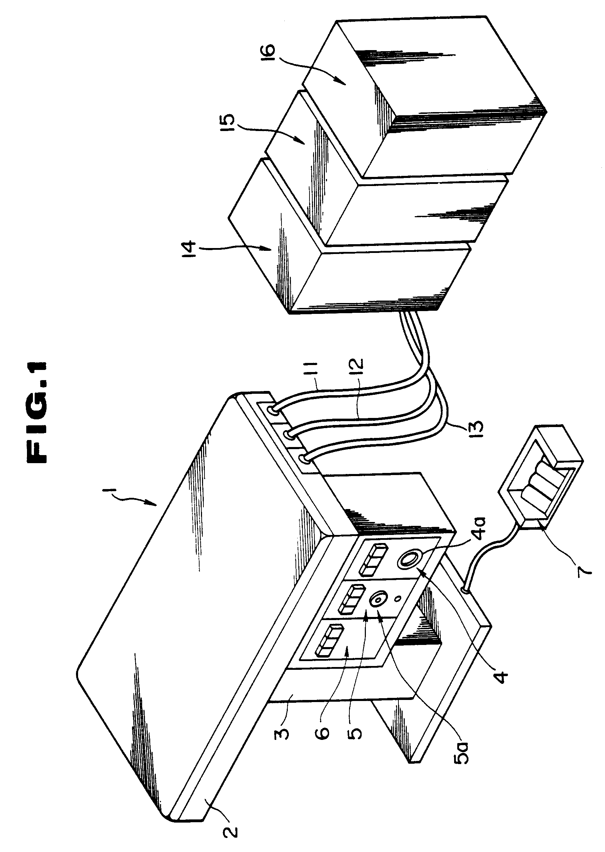

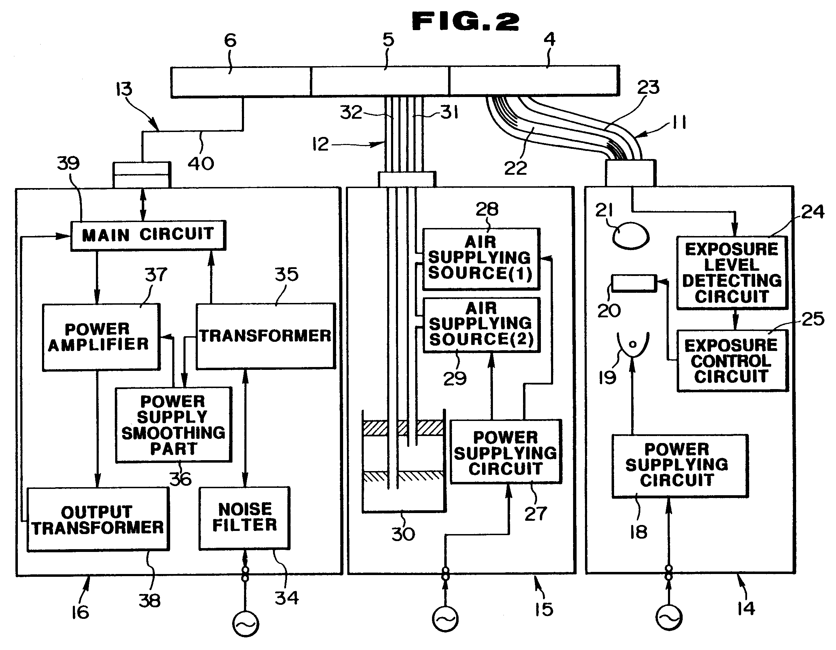

Figs. 1 and 2 relate to the first embodiment of this invention.

Fig. 1 is a perspective view showing a bed and peripheral devices.

Fig. 2 is a block diagram showing the construction of peripheral devices.

### Detailed Description of the Preferred Embodiments

Figs. 1 and 2 show a preferred embodiment of this invention.

As shown in Fig. 1, an endoscope examination apparatus is provided with an endoscope examination bed 1 which comprises a bed body 2 on which a subject lies, a supporting part 3 for supporting the bed body 2, a light source control part 4 comprising three control means and an operating means attached to the bottom of said bed body 2, an air and water supplying device control part 5 and a high frequency cautery device control part 6. An illuminating light emitting end part 4a is provided in said light source device control part 4. An air and water supplying channel connection part 5a is provided in said air and water supplying device control part 5. A light guide connector of an endoscope can be connected to said illuminating light emitting end part 4a. An air and water supplying channel of an endoscope is connected to the air and water supplying channel connection part 5a. Also, a foot switch 7 is connected to the bottom of said supporting part 3.

To the side of said bed body 2, a light source device 14, an air and water supplying device 15 and a high frequency cautery device 16, said three devices being used for an endoscope examination are connected through cables 11, 12 and 13, respectively, and said three cables comprises the connecting means.

As shown in Fig. 2, the light source device control part 4, the air and water supplying device control part 5 and the high frequency cautery device control part 6 are connected to the light source device 14, the air and water supplying device 15 and the high frequency cautery device 16 through said cables 11, 12 and 13, respectively.

Said light source device 14 comprises a power supplying circuit 18 connected with an outer power supply and a lamp 19 supplied with electric power from the power supplying circuit 18. The light emitted from said lamp 19 is converged by being passed through an iris 20 and through a condenser 21, and enters an incident end of a light guide 22 inserted in the cable 11. An emitting end of the light guide 22 is connected to the illuminating light emitting end part 4a in said light source device control part 4. Also, in said cable 11, a cord 23 connecting the light source device control part 4 and a circuit in the light source device 14 is inserted. Then, through the cord 23, the light source device 14 can be turned on and off and the amount of light can be controlled by the light source device control part 4. Also, the signal from a light amount detecting means, which is not illustrated, provided in the endoscope is fed to an exposure level detecting circuit 24 provided in the light source device 14 through said cord 23. The output of the exposure level detecting circuit 24 is fed to an exposure control circuit 25 for driving said iris 20. Then, according to the exposure level detected by the exposure level detecting circuit 24, the amount of light is automatically adjusted by the iris 20 controlled by the exposure control circuit 25.

Said air and water supplying device 15 is provided with a power supplying circuit 27 connected with an outer power supply, an air supplying source (1)28 and an air supplying source (2)29 connected with the power supplying circuit 27 and a tank 30 for storing water. Also, in the cable 12, an air supplying channel 31 connected to said air and water supplying channel connection part 5a and a water supplying channel 32 are included. The air supplying channel 31 is connected to said air supplying source (1)28, said air supplying source (2)29 and the upper space of said tank 30. Also, said water supplying channel 32 is connected to said tank 30. Then, by controlling said air supplying sources 28 and 29, air or water can be supplied through the air supplying channel 31 or the water supplying channel 32, respectively. Said air supplying sources 28 and 29 can be controlled by the air and water supplying device control part 5 through a cord, which is not illustrated, included in the cable 12.

Said high frequency cautery device 16 is provided with a noise filter 34 connected with the outer power supply, a transformer 35 connected with the noise filter 34, a power supply smoothing part 36 connected with the transformer 35, a power amplifier 37 connected with the power supply smoothing part 36, an output transformer 38 connected with the power amplifier 37 and a main circuit 39 connected with the high frequency cautery device control part 6 through a cord 40 included in the cable 13. Said main circuit 39 may be connected with an electrode which is not illustrated. The electrode is supplied with a cautery current from the outer power supply through the noise filter 34, the transformer 35, the power supply smoothing part 36, the power amplifier 37, the output transformer 38 and the main circuit 39. The main circuit 39 switches the output wave form of said cautery current and controls the power amplifier 37 so as to control the output. The main circuit 39 is controlled by the high frequency cautery device control part 6. Further, the main circuit 39 is supplied with electric power from the transformer 35.

Also, the foot switch 7 is connected with the main circuit 39 in said high frequency cautery device 16 and the cautery current can be also controlled by the foot switch 7.

Thus, the light source device 14, the air and water supplying device 15 and the high frequency cautery device 16 comprising the endoscope peripheral devices used for an endoscope examination are positioned apart from the bed 1, and the control parts 4, 5 and 6 of said devices 14, 15 and 16, respectively, are provided in the bed 1. Therefore, according to the present invention, the bed 1 does not become larger and electric devices are not dirtied by the body fluid, dirt or the like of a patient, and also the endoscope peripheral devices can be easily controlled.

Further, in addition to the control parts 4, 5 and 6 provided in the bed 1, the illuminating light emitting end part 4a at which an endoscope is connected to the light source device 14 and the air and water supplying channel connection part 5a of the air and water supplying device 15 are provided in the bed 1 so that it is not necessary for the devices 14, 15 and 16 to be placed on the same side of the bed as of the operator. Therefore, these devices do not become obstacles to the operation.

Also, since the cables 11 to 13 comprising the connecting means extend from another side of the bed body 2 and are connected to the devices 14 to 16, these cables 11 to 13 do not become obstacles.

Also, the endoscope peripheral devices are not limited only to the devices shown in the embodiment, but may also be an electronic endoscope light source device, a video processor (signal processing device) and a suction device.

Further, in this embodiment, a single unit comprising the control parts of each device and a connecting part to the endoscope is provided in the bed.

As explained above, according to this embodiment, it is advantageous that endoscope peripheral devices are not included in the bed and also that said devices can be easily controlled because a control means for controlling endoscope peripheral devices which are separated from an endoscope examination bed is provided.

## Claims

1. An endoscope examination apparatus provided with a bed (1) on which a subject can lie, comprising:
a device (14, 15, 16) positioned separately from said bed (1) and used for an endoscope examination;
a control means (4, 5, 6) provided in said bed (1) for controlling said device (14, 15, 16);
a connecting means (11, 12, 13) for operatively connecting said device (14, 15, 16) and said control means (4, 5, 6) **characterized** in
that said control means (4, 5, 6) are fixed by arranged in the bed (1) and acsessible from one side thereof and in that the said connecting means (11, 12, 13) are connecting said device (14, 15, 16) and said control means (4, 5, 6) via a part arranged on another side of said bed (1).

2. An endoscope examination apparatus according to claim 1, wherein said at least one device comprises:
a light source device (14),
an air and water supplying device (15), and
a high frequency cautery device (16).

## Patentansprüche

1. Ein endoskopisches Untersuchungsgerät, das mit einem Bett (1) ausgestattet ist, auf dem eine Person liegen kann, umfassend:
eine Einrichtung (14, 15, 16), die von dem Bett (1) getrennt angeordnet ist und für eine endoskopische Untersuchung verwendet wird;
ein in dem Bett (1) vorgesehenes Steuermittel (4, 5, 6) zur Steuerung der Einrichtung (14, 15, 16);
ein Anschlußmittel (11, 12, 13), um die Einrichtung (14, 15, 16) und das Steuermittel (4, 5, 6) betriebsbereit zu verbinden;
dadurch gekennzeichnet,
daß die Steuermittel (4, 5, 6) unbeweglich in dem Bett (1) angeordnet und von einer Seite desselben zugänglich sind und daß die Anschlußmittel (11, 12, 13) die Einrichtung (14, 15, 16) und die Steuermittel (4, 5, 6) über einen Teil, der auf einer anderen Seite des Betts (1) angeordnet ist, verbinden.

2. Ein endoskopisches Untersuchungsgerät nach Anspruch 1, wobei die mindestens eine Einrichtung umfaßt:
eine Beleuchtungseinrichtung (14),
eine Luft- und Wasserzufuhreinrichtung (15) und
eine Hochfrequenz-Kauterisationseinrichtung (16).

## Revendications

1. Appareil d'examen par endoscopie prévu avec un lit (1) sur lequel un sujet peut s'étendre, comprenant :
un dispositif (14, 15, 16) positionné séparément dudit lit (1) et utilisé pour un examen par endoscopie,
un moyen de commande (4, 5, 6) prévu dans ledit lit (1) destiné à commander ledit dispositif (14, 15, 16),
un moyen de connexion (11, 12, 13) destiné à connecter de façon fonctionnelle ledit dispositif (14, 15, 16) et ledit moyen de commande (4, 5, 6)
caractérisé en ce que
ledit moyen de commande (4, 5, 6) est agencé de façon fixe dans le lit (1) et est accessible depuis un côté de celui-ci, et en ce que ledit moyen de connexion (11, 12, 13) connecte ledit dispositif (14, 15, 16) et ledit moyen de commande (4, 5, 6) par l'intermédiaire d'une partie agencée sur un autre côté dudit lit (1).

2. Appareil d'examen par endoscopie selon la revendication 1, dans lequel ledit au moins un dispositif comprend :
un dispositif de source de lumière (14),
un dispositif d'alimentation en air et en eau (15), et
un dispositif de cautérisation à haute fréquence (16).
